# EUROPÄISCHE PATENTANMELDUNG

(11) **EP 3 878 387 A1**
(43) Veröffentlichungstag der Anmeldung: **15.09.2021**
(21) Anmeldenummer: 20162460.8
(22) Anmeldetag: 11.03.2020
(51) Int. Cl.: A61B 17/86, A61B 17/80

(54) **SCHRAUBE, SYSTEM MIT EINER SCHRAUBE UND EINER PLATTE, SOWIE VERFAHREN ZUM HERSTELLEN EINER SCHRAUBE**

(71) Anmelder: Medartis Holding AG, 4057 Basel (CH)
(72) Erfinder: Mullis, Andreas, 4465 Hemmiken (CH); Thiel, Dirk, 79219 Staufen (DE); Sesiani, Patrick Mark, 79739 Schwörstadt (DE); Würger, Stefan Olivier, 4415 Lausen (CH); Schonhardt, Jürgen, 79618 Rheinfelden (DE); Zeuner, Hermann, 79111 Freiburg (DE)
(74) Vertreter: Hepp Wenger Ryffel AG

(57) **Zusammenfassung**

Die Erfindung betrifft Schraube (1), bevorzugt eine Knochenschraube, die einen Schaft (2) mit einer Spitze (3), einen Kopf (4) und ein Gewinde (5) umfasst. Der Schaft umfasst weiter eine Längsachse (A). Eine Schar von Radialquerschnittsflächen (Q) des Kopfes (4) verlaufen durch die Längsachse (A) der Schraube (1), wobei die Lage jeder Radialquerschnittsfläche (Q) durch einen Azimutwinkel (6) in einer Ebene (E) senkrecht zur Längsachse (A) definiert ist. Jede Radialquerschnittsfläche (Q) hat einen Flächeninhalt, welcher durch die Längsachse (A) und die Aussenfläche der Schraube (8) definiert wird. Das Gewinde (5) erstreckt sich derart in den Kopf (4), dass die Flächeninhalte der Radialquerschnittsflächen (Q') in einem ersten Azimutwinkelbereich (9) konstant sind und die Flächeninhalte der Radialquerschnittsflächen (Q") in einem zweiten, vom ersten verschiedenen Azimutwinkelbereich (10) einen abweichenden, bevorzugt kleineren, Wert aufweisen als die Flächeninhalte im ersten Azimutwinkelbereich (9). Der der erste Azimutwinkelbereich (9) beträgt höchstens 350°, bevorzugt höchstens 345°.

## Beschreibung

Die Erfindung betrifft eine Schraube, ein System umfassend eine Schraube und eine Platte, sowie ein Verfahren zum Herstellen einer Schraube gemäss dem Oberbegriff der unabhängigen Patentansprüche.

Es ist bekannt, Schrauben zum Verbinden von Implantaten mit Knochen zu verwenden. Beispielsweise werden bestimmte Knochenbrüche behandelt, indem die gebrochenen Knochen mittels einer Knochenplatte verbunden und mit Schrauben fixiert werden.

Bekannte Schrauben weisen diverse Nachteile auf. So sind herkömmliche Schrauben häufig nicht anwendbar, wenn der zu verbindende Knochen eine dünne Kortikalisschicht aufweist, da die Schraube dann nicht ausreichend in den Knochen eingreifen kann. Ebenso können herkömmliche Schrauben bei der Anwendung unter dünnen Gewebeschichten ästhetisch nachteilig sein, weil zur sicheren Verankerung im Knochen dicke Platten erforderlich sind.

Es ist daher Aufgabe der vorliegenden Erfindung, die Nachteile des Bekannten zu überwinden und insbesondere eine Schraube zur Verfügung zu stellen, die eine grössere Flexibilität in der Anwendung bietet. Beispielsweise soll sich die Schraube auch mit dünnen Platten und/oder zur Verbindung mit dünnen Knochen eignen und so ein grösseres Anwendungsspektrum ermöglichen.

Diese und weitere Vorteile werden durch eine Schraube, ein System umfassend eine Schraube und eine Platte, sowie ein Verfahren zum Herstellen einer Schraube gemäss dem kennzeichnenden Teil der unabhängigen Ansprüche erzielt.

Die erfindungsgemässe Schraube ist bevorzugt eine Knochenschraube und umfasst einen Schaft mit einer Spitze, einen Kopf und ein durchgehendes Gewinde, sowie eine Längsachse des Schaftes. Weiter umfasst die Schraube eine Schar von Radialquerschnittsflächen des Kopfes, die durch die Längsachse des Schaftes begrenzt werden. Die Lage jeder Radialquerschnittsfläche ist durch einen Azimutwinkel in einer Ebene senkrecht zur Längsachse definiert. Jede Radialquerschnittsfläche umfasst einen Flächeninhalt, welcher durch die Längsachse und die Aussenfläche der Schraube definiert wird. Das Gewinde erstreckt sich erfindungsgemäss derart in den Kopf, dass die Flächeninhalte der Radialquerschnittsflächen in einem ersten Azimutwinkelbereich konstant sind. Die Flächeninhalte der Radialquerschnittsflächen in einem zweiten Azimutwinkelbereich weisen einen von den Flächeninhalten des ersten Azimutwinkelbereiches einen abweichenden, bevorzugt kleineren, Wert auf. Der erste Azimutwinkelbereich ist vom zweiten Azimutwinkelbereich verschieden. Der erste Azimutwinkelbereich beträgt höchstens 350°, bevorzugt höchstens 345°.

Dadurch erstreckt sich das Gewinde näher an den Schraubenkopf als bei herkömmlichen Schrauben und insbesondere bis in den Kopf. Daher kann die Schraube auch bei der Verwendung von dünnen Platten und/oder bei der Behandlung von Knochen mit dünnen Kortikalisschichten in den Knochen eingreifen.

Ein Radialquerschnitt des Kopfes soll verstanden werden als der Teil der gesamten Querschnittsfläche des Kopfes, der auf der einen Seite der Längsachse liegt und durch die Längsachse begrenzt ist. Ein Querschnitt des Kopfes weist daher definitionsgemäss zwei Radialquerschnitte auf.

Ein durchgehendes Gewinde ist so ausgeführt, dass das Gewinde einen Anfangs- und einen Endpunkt aufweist und dazwischen keine gewindefreien Abschnitte angeordnet sind. Insbesondere weist ein durchgehendes Gewinde zwischen dem Anfangs- und Endpunkt eine konstante Gewindesteigung auf. Ein durchgehendes Gewinde kann mehrgängig sein.

Die Schraube weist daher bevorzugt genau ein durchgehendes Gewinde auf. Dieses kann sich insbesondere von der Spitze bis in den Kopf erstrecken. Der Gewindeabschnitt, der sich in den Kopf erstreckt, kann insbesondere auch nur als Stumpf ausgeführt sein.

Insbesondere bei der Verwendung und Fixierung von Knochenplatten mit einer Dicke von 0.1 bis 1 mm ist die erfindungsgemässe Schraube vorteilhaft. Bei der Behandlung von Knochen mit dünner Weichteilabdeckung sind solche dünnen Platten besonders vorteilhaft. Aber auch bei Knochen mit dünner Kortikalis, insbesondere in einem Bereich von 0.1 bis 2 mm und/oder schlechter Spongiosaqualität und/oder bei monokortikaler Verankerung bietet die erfindungsgemässe Schraube sichereren Halt im Vergleich zum Stand der Technik.

Die Flächeninhalte umfassen nur die Fläche der Radialquerschnittsfläche auf einer Seite der Längsachse. Jede Querschnittsfläche umfasst daher zwei Radialquerschnittsflächen mit je einem Flächeninhalt, deren Lage durch einen um 180° verschiedenen Azimutwinkel definiert ist.

Insbesondere kann der Azimutwinkel, der die Lage der jeweiligen Flächeninhalte definiert, als relative Angabe zu einem willkürlichen Nullpunkt verstanden werden. Bevorzugt wird als Nullpunkt das Ende des Gewindes verwendet. In diesem Fall ist der Flächeninhalt mit einem Azimutwinkel von 0° definitionsgemäss dem ersten Azimutwinkelbereich zugeordnet, während der direkt angrenzende Flächeninhalt (mit einem Azimutwinkel grösser als 359°) definitionsgemäss zum zweiten Azimutwinkelbereich gehört. Dieses Beispiel dient lediglich dem besseren Verständnis der Beschreibung der Erfindung. Es ist analog möglich, einen belieben Nullpunkt zur Definition der Lage der Flächeninhalte zu wählen.

Zur Berechnung der Flächeninhalte sollen nicht-gewindebezogene Konturen ausgeschlossen werden. Beispielsweise kann im Schraubenkopf ein Antrieb (wie Torx, Phillips, Schlitz) angeordnet sein, der die Flächeninhalte der Radialquerschnittsflächen mangels Rotationssymmetrie ungleichmässig verändern würde. In diesem Fall kann der Antrieb gedanklich ausgefüllt werden, so der Antrieb für die Berechnung der Flächeninhalte keine Bedeutung hat.

Bevorzugt kann der Flächeninhalt in einer Richtung der Längsachse in Richtung des Schraubenkopfes auch nur bis zum Äquator mit einbezogen werden, also bis zur breitesten Stelle des Schraubenkopfes in einer Ebene senkrecht zur Längsachse.

Der Kopf der Schraube kann insbesondere als ein Bereich der Schraube entlang der Längsachse verstanden werden, der sich in einer Ebene senkrecht zur Längsachse in radialer Richtung zumindest teilweise über das Gewinde, insbesondere einen Aussenradius des Gewindes, erstreckt. Dementsprechend können die Flächeninhalte der Radialquerschnitte auch so berechnet werden, dass der Bereich der Schraube, der der obigen Definition entsprechend nicht zum Kopf gehört, nicht mit einbezogen werden.

Die Schraube kann ein Material aus der Gruppe Titan, Titanlegierungen und Implantatstahl umfassen. Es sind aber auch andere biokompatible Materialien denkbar. Bevorzugt besteht die Schraube aus einem der genannten Materialien.

Die Schraube kann bevorzugt ein Gewinde mit einer Gewindesteigung von 0.4 bis 0.6 mm bei einem Gewindedurchmesser von 0.9 oder 1.2 mm aufweisen.

Alternativ kann die Schraube auch eine Gewindesteigung von 0.5 bis 0.75 mm bei einem Gewindedurchmesser von 1.5 oder 1.8 mm aufweisen.

Andere Gewindedurchmesser und Gewindesteigungen sind aber denkbar, insbesondere Gewindedurchmesser von 0.5 bis 2.5 mm und Gewindesteigungen von 0.1 bis 1.5 mm.

Bevorzugt weist die Schraube einen Antrieb ausgewählt aus einer Gruppe umfassend Schlitz, Kreuz, Mehrkant, Torx und Phillips auf.

Bei zwei-, drei- oder mehrgängigen Schrauben können der erste und der zweite Azimutwinkelbereich in mindestens zwei, insbesondere in zwei oder drei oder mehr, Abschnitte unterteilt sein, die in gleichen Azimutwinkelabständen verteilt sind.

Dies bedeutet, dass der komplette Umfangswinkelbereich in die entsprechend mehrfache Anzahl Abschnitte unterteilt ist. Die ersten und zweiten Azimutwinkelbereiche wechseln sich diesfalls ab. Wenn der erste und zweite Azimutwinkel beispielsweise in zwei Abschnitte unterteilt sind, sind die beiden Abschnitte des ersten Azimutwinkelbereiches jeweils durch die Abschnitte des zweiten Azimutwinkelbereiches begrenzt.

Insbesondere kann der Azimutwinkelabstand von der Mitte der jeweiligen Abschnitte gemessen werden und beträgt diesfalls bevorzugt 180° oder 120°.

Der erste Azimutwinkelbereich kann höchstens auch 330°, bevorzugt höchstens 305°, betragen.

Der angegebene Azimutwinkelbereich ist als die Summe der einzelnen Abschnitte der jeweiligen Azimutwinkelbereiche zu verstehen.

Das Gewinde der Schraube kann mehrgängig, bevorzugt zwei oder dreigängig sein.

Der erste Azimutwinkelbereich kann, insbesondere bei mehrgängigen Gewinden, auch höchstens 340°, 320°, 315°, oder 300° betragen. Bei mehrgängigen Gewinden mit n Gängen beträgt der zweite Azimutwinkelbereich besonders bevorzugt mindestens n x 15°. Der erste Azimutwinkelbereich beträgt in diesem Fall daher höchstens die Differenz von 360° und n x 15°.

Wenn das Gewinde mehrgängig ist, kann der Azimutwinkelbereich in gleich viele Abschnitte unterteilt sein, wie das Gewinde Gänge aufweist.

Bevorzugt weist der Kopf im zweiten Azimutwinkelbereich mindestens eine Einkerbung auf, die durch die Fortsetzung des Gewindes gebildet ist.

Die Einkerbung kann insbesondere durch ein Wirbelmesser, das zur Herstellung des Gewindes verwendet wird, erzeugt sein. Die Einkerbung kann ein tieferes Einschrauben in den Knochen, insbesondere in den harten kortikalen Knochen unmittelbar unter der Platte, ermöglichen, bevor der Schraubenkopf beispielsweise auf einer Knochenplatte aufliegt. Dadurch wird die Versorgung stabiler, weil Gewindeanteile knapp unterhalb des Kopfes noch im Knochen verankert werden können, und Beschädigungen des Knochens können reduziert werden.

Der Kopf kann eine Anzahl solcher Einkerbungen aufweisen, die der Anzahl Abschnitte entspricht.

Bevorzugt weist jeder Abschnitt des zweiten Azimutwinkelbereiches mindestens eine Einkerbung auf, welche durch die Fortsetzung des Gewindes gebildet ist, auf.

Die Einkerbung kann insbesondere stumpfförmig ausgebildet sein und kann auch einen mindestens teilweise elliptischen Bereich aufweisen. Besonders bevorzugt weist die Einkerbung in Umfangsrichtung unterschiedliche Breiten auf, wobei die Dicke in Richtung des Gewindelaufes zunächst zunimmt und in einem Endabschnitt wieder abnimmt. Die maximale Breite der Einkerbung kann in diesem Fall in einem Bereich angeordnet sein, wo das Gewinde im Schaft endet.

Die mindestens eine Einkerbung, welche durch die Fortsetzung des Gewindes gebildet ist, kann an einer Unterseite des Kopfes im Bereich des Gewindeauslaufes angeordnet sein.

Die Unterseite des Kopfes kann insbesondere verstanden werden als der Bereich der Oberfläche des Kopfes, deren Flächennormale zumindest teilweise in Richtung der Spitze des Schraubenschaftes zeigt, insbesondere bezogen auf eine Ebene senkrecht zur Längsachse. Die Unterseite des Kopfes umfasst den Bereich der Oberfläche des Kopfes, die vom Äquator des Kopfes aus gesehen auf der Seite der Spitze des Schaftes angeordnet ist.

Die Radialquerschnittsflächen weisen einen Flächenschwerpunkt auf. Ein Flächenschwerpunkt ist dabei gemäss gängiger Definition zu verstehen. Der Flächenschwerpunkt der Radialquerschnittsflächen im ersten Azimutwinkelbereich kann jeweils den gleichen Abstand zur Längsachse aufweisen. Bevorzugt weist der Flächenschwerpunkt der Radialquerschnittsflächen im ersten Azimutwinkelbereich jeweils eine identische Lage bezogen auf die Längsachse auf.

Besonders bevorzugt weisen die Radialquerschnittsflächen im ersten Azimutwinkelbereich zusätzlich eine identische Form auf. Besonders bevorzugt weisen die Radialquerschnitte innerhalb des zweiten Azimutwinkelbereiches unterschiedliche Formen auf. Es ist aber denkbar, dass die Radialquerschnittsflächen im zweiten Azimutwinkelbereich ebenfalls eine identische Form aufweisen. Selbst in diesem Fall wären aber die Flächeninhalte der Radialquerschnitte im zweiten Azimutwinkelbereich kleiner als im ersten Azimutwinkelbereich.

Die Schraube kann so ausgebildet, insbesondere geformt, sein, so dass jede Radialquerschnittsfläche zusammenhängend, insbesondere wegzusammenhängend oder einfach zusammenhängend, ist.

Eine Fläche ist wegzusammenhängend, wenn zwei beliebige Punkte der Fläche durch einen Weg innerhalb der Fläche verbunden werden können.

Eine Fläche ist einfach zusammenhängend, wenn er wegzusammenhängend ist und eine beliebige geschlossene Linie sich auf einen Punkt zusammenziehen lässt.

Insbesondere umfasst der Schraubenkopf dadurch keine durchgehenden Bohrungen oder Löcher. Insbesondere umfasst der Schraubenkopf bevorzugt keine Bohrungen oder Löcher, die die Längsachse mit der Oberfläche der Schraube oder des Kopfes verbinden.

Die Schraube, insbesondere der Kopf der Schraube, kann einen ersten Normalquerschnitt in einer Ebene senkrecht zur Längsachse im Kopfbereich der Schraube aufweisen, der nicht kreisrund ist.

Bevorzugt weist die Schraube, insbesondere der Kopf der Schraube, einen zweiten Normalquerschnitt auf, der kreisförmig ist und parallel zum ersten Normalquerschnitt ist. Der erste Normalquerschnitt ist dabei bevorzugt entlang der Längsachse der Schraube zwischen der Spitze der Schraube und dem zweiten Normalquerschnitt angeordnet.

Die Erfindung bezieht sich weiterhin auf ein System, umfassend eine Knochenplatte und eine Knochenschraube. Insbesondere kann das System eine Knochenschraube mit den vorgehend beschriebenen Merkmalen umfassen. Die Knochenplatte umfasst mindestens eine Öffnung zum Einsetzen der Knochenschraube. Die Knochenschraube umfasst einen Kopf, einen Schaft und ein durchgehendes Gewinde. Die Knochenplatte hat bevorzugt eine Dicke, die höchstens der Länge des Kopfes der Schraube entlang einer Längsachse der Schraube entspricht. Insbesondere kann die Dicke der Platte einen Wert zwischen 0.1 bis 1 mm aufweisen. Die Knochenschraube ist derart in die Öffnung der Knochenplatte einsetzbar, dass der Kopf in mechanischem Kontakt mit der Knochenplatte ist, insbesondere auf der Knochenplatte aufliegt, und das Gewinde sich zumindest teilweise in die Öffnung hinein erstreckt.

Die Erfindung bezieht sich weiterhin auf ein Verfahren zum Herstellen einer Schraube, insbesondere einer Knochenschraube. Bevorzugt wird das Verfahren angewendet, um eine vorgehend beschriebene Schraube herzustellen. Die Schraube wird nach dem erfindungsgemässen Verfahren mit einem Werkzeug, insbesondere ausgewählt aus einer Gruppe umfassend Wirbelmesser und Gewindefräser, hergestellt. Es sind aber auch andere, vergleichbaren Werkzeuge denkbar. Das Verfahren umfasst das Bereitstellen eines Rohlingmaterials mit einer im Wesentlichen zylindrischen Form sowie das Schneiden eines Gewindes entlang einer Längsachse der zylindrischen Form mit dem Werkzeug. Es wird ausserdem ein Kopf geformt, insbesondere gedreht. Die Schraube wird anschliessend mittels einer Konturspannzange abgegriffen. Die Konturspannzange weist eine Kontur auf, die einer Kontur des Kopfes bzw. Teilen des Kopfes angepasst ist und die im abgegriffenen Zustand mit einer Gegenkontur des Kopfes bzw. Teilen des Kopfes in Wirkverbindung steht. Es ist optional auch möglich, zusätzlich zumindest teilweise auch an Teilen des Schaftes, des Halses oder des Gewindes abzugreifen.

Dadurch kann die Schraube abgegriffen werden, ohne das Gewinde, das sich bis in den Schraubenkopf erstreckt, zu beschädigen.

Bevorzugt wird zuerst ein Kopf in das Rohlingmaterial gedreht, und danach das Gewinde geschnitten.

Bevorzugt ist die Kontur der Konturspannzange zumindest teilweise im Wesentlichen komplementär zur Gegenkontur des Kopfes bzw. von Teilen des Kopfes sowie Teilen des Halses oder Gewindes der Schraube ausgeführt. Dadurch passt die Kontur zumindest teilweise genau auf die Gegenkontur, wodurch ein besonders sicheres Halten ermöglicht wird.

Bevorzugt wird das Gewinde mit Hilfe des Werkzeuges so nahe an den Kopf geschnitten, dass das Werkzeug eine Einkerbung an einer Unterseite des Kopfes bildet. Dies wird insbesondere dann so ausgeführt, wenn der Kopf gedreht wird, bevor das Gewinde geschnitten wird. Es ist jedoch auch denkbar, dass die Form des Kopfs erst nach dem Schneiden des Gewindes gedreht wird.

Bevorzugt wird ein Werkzeug verwendet, das eine Wirbelplatte aufweist, wobei die Wirbelplatte in einer Schneidrichtung an der führenden Fläche, also Richtung des Schraubenkopfes und weg vom bereits geschnittenen Gewinde, keine Überschneide umfasst. Die Wirbelplatte kann daher tiefer in den Schraubenkopf eindringen, ohne einen Abdruck der Überschneide zu hinterlassen.

Bevorzugt wird nach dem Schneiden des Gewindes mit dem Werkzeug der Kopf mittels einer CNC Langdrehmaschine weiterbearbeitet. Insbesondere kann ein Schraubenantrieb, beispielsweise ein Torx-Antrieb, gefertigt werden.

Die Erfindung wird im Folgenden anhand der folgenden Figuren im Detail erläutert. Es zeigen:
- Fig. 1:: eine Schraube in einer Seitenansicht.
- Fig. 2a-2c:: ein Schraubenkopf einer Schraube in einer Seitenansicht, perspektivischen Sicht, und einer Untersicht.
- Fig. 3a-3c:: Querschnitte eines Schraubenkopfes in den Ebenen E, E' und E" von Fig. 2c.
- Fig. 4:: eine Untersicht eines Kopfes einer alternativen Ausführungsform einer Schraube.
- Fig. 5:: eine Knochenplatte mit einer Schraube.
- Fig. 6:: eine alternative Schraube in einer Seitenansicht.
- Fig. 7:: schematisch einen Verfahrensschritt zur Herstellung einer Schraube.

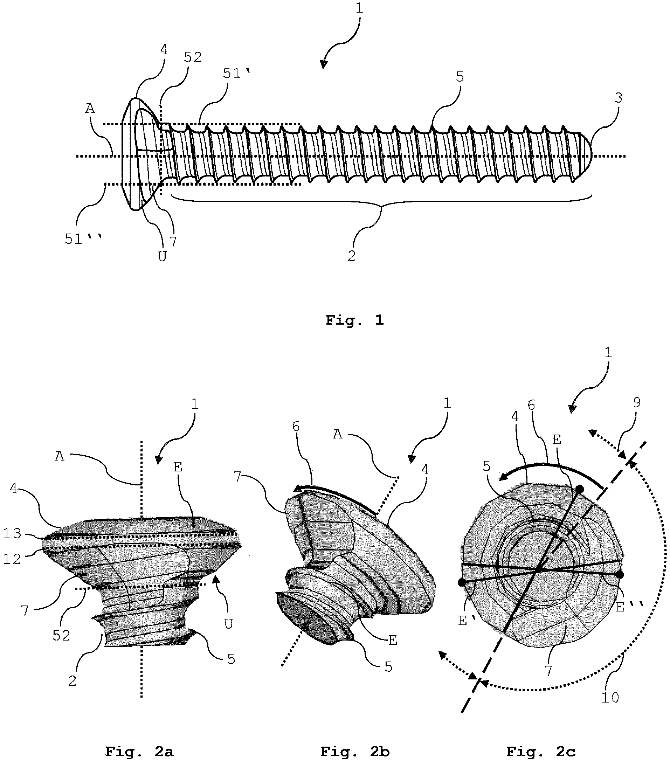

Fig. 1 zeigt eine erfindungsgemässe Schraube 1 in einer Seitenansicht. Die Schraube 1 umfasst einen Schaft 2 mit einer Spitze 3, ein Gewinde 5, sowie einen Kopf 4. Vorliegend ist der Kopf 4 begrenzt durch eine Ebene 52, die senkrecht zur Längsachse A der Schraube 1 liegt. Die Ebene 52 ist entlang der Längsachse A so positioniert, dass der Schaft 2 der Schraube 1 in distale Richtung der Spitze 3 (in der Fig. 1 rechts von der Ebene 52) im Querschnitt senkrecht zur Längsachse nirgends über den Radius des Aussengewindes 51',51" hinausragt. Ein Abschnitt in die andere proximale Richtung (in der Darstellung von Fig. 1 links der Ebene 52) ragt dagegen über den Radius des Aussengewindes 51',51" hinaus und definiert den Kopf 4 der Schraube. Das Gewinde 5 erstreckt sich über die Ebene 52 hinaus in den Kopf und bildet an der Unterseite U des Kopfes 4 eine Einkerbung 7.

Fig. 2a zeigt den Kopf 4 der erfindungsgemässen Schraube 1 in einer Seitenansicht. Zum besseren Verständnis ist ein Teil des Schaftes 2 mit dem Gewinde 5 ebenfalls gezeigt. Die Ebene 52 trennt den Kopf 4 vom Schaft 2. Das Gewinde 5 erstreckt sich über die Ebene 52 hinaus in den Kopf und bildet an der Unterseite des Kopfes eine Einkerbung. Der Schraubenkopf 4 umfasst daher einen ersten Normalquerschnitt 12 sowie einen zweiten Normalquerschnitt 13. Der erste Normalquerschnitt 12 schneidet die Einkerbung 7 und ist daher nicht kreisförmig. Der zweite Normalquerschnitt 13 ist entlang der Längsachse weiter von der Schraubenspitze 3 entfernt als der erste Normalquerschnitt 12. Es ergibt sich daher, dass der erste Normalquerschnitt 12 entlang der Längsachse A zwischen dem zweiten Normalquerschnitt 13 und der Spitze 3 angeordnet ist. Da der zweite Normalquerschnitt 13 über der Einkerbung 7 liegt, weist der zweite Normalquerschnitt eine Kreisform auf. Eine Ebene E, die koplanar mit der Längsachse A ist, grenzt vorliegend an die Einkerbung 7.

Fig. 2b zeigt den Schraubenkopf 4 aus Fig. 2a in einer perspektivischen Ansicht. Ein Azimutwinkel 6 ist vorliegend in einer Gegenuhrzeigerrichtung um die Längsachse A eingezeichnet, wobei die Ebene E vorliegend und für die nachfolgenden Figuren als Nullpunkt dient. Wie oben ausgeführt könnte ein beliebiger anderer Nullpunkt gewählt werden. Ebenso wäre es möglich, den Azimutwinkel 6 im Uhrzeigersinn zu messen.

Fig. 2c zeigt den Schraubenkopf 4 aus den Figuren 2a und 2b in einer Untersicht. Die Längsachse A, hier nicht gezeigt, verläuft senkrecht zur Bildebene, so dass der Azimutwinkel 6 entsprechend in der Bildebene liegt. Ein erster Azimutwinkelbereich 9 weist keine Einkerbung 7 auf und erstreckt sich über einen Azimutwinkel von 195°. Die Einkerbung 7 erstreckt entsprechend über einen Azimutwinkel von 165° und definiert einen zweiten Azimutwinkelbereich 10. Der zweite Azimutwinkelbereich 10 hat daher einen Wert von 165°. Aufgrund der Einkerbung 7 sind die Querschnitte des Schraubenkopfes 4 in Ebenen durch die Längsachse, die zumindest teilweise im zweiten Azimutwinkelbereich 10 liegen, nicht spiegelsymmetrisch zur Längsachse A. Die Radialquerschnitte, die durch die Längsachse A auf der einen Seite und durch die Aussenfläche des Kopfes 4 auf der anderen Seite begrenzt werden, haben im zweiten Azimutwinkelbereich 10 einen kleineren Flächeninhalt als im ersten Azimutwinkelbereich 9. Dieses Merkmal wird nachfolgend anhand von Querschnittsfiguren in den hier dargestellten Ebenen E, E' und E" beschrieben. Die Ebene E befindet sich wie vorgängig erwähnt in einem Azimutwinkel 6 von 0°, die Ebene E' in einem Azimutwinkel 6 von 135° und die Ebene E" in einem Azimutwinkel 6 von 300°. Alle Ebenen sind hier auf der einen Seite mit einem Punkt versehen, das in den nachfolgenden Figuren das Verständnis der Ebenenorientierung erleichtern soll.

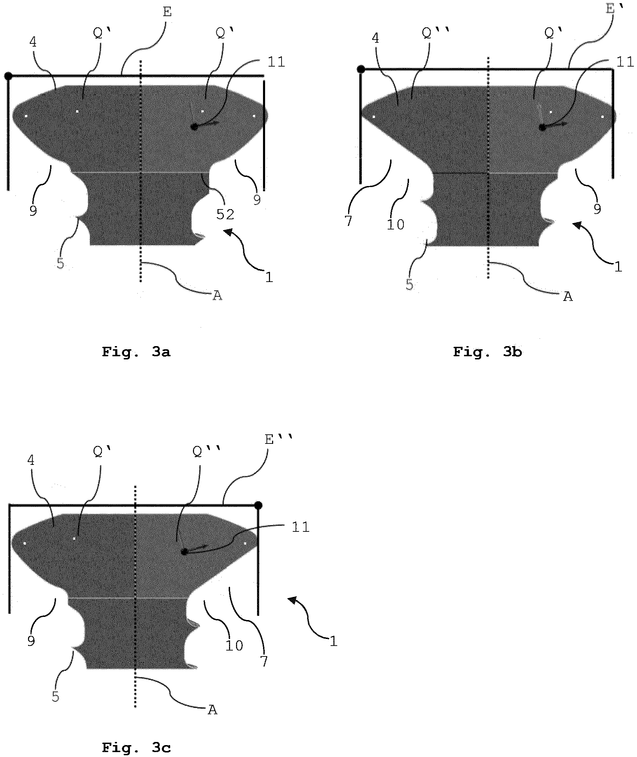

Fig. 3a zeigt eine Querschnittsansicht des Schraubenkopfes der Figuren 2a-2c in der Ebene E. Der Kopf 4 ist durch die Ebene 52 begrenzt, so dass nur die Fläche innerhalb der Umrandung des Kopfes 4 und der Ebene 52 für die nachfolgende Diskussion der Flächeninhalte miteinbezogen wird. Zwei Radialquerschnitte Q' sind jeweils von der Umrandung des Kopfes 4, der Längsachse A und der Ebene 52 begrenzt und weisen Flächeninhalte Q' auf. Die Ebene E schneidet die Kerbe 7 (hier nicht gezeigt) nicht. Daher sind beide Flächeninhalte Q' identisch und befinden sich im ersten Azimutwinkelbereich 9. Der Querschnitt ist bezogen auf die Längsachse A spiegelsymmetrisch. Daher weisen die beiden Radialquerschnitte Q' die gleiche Form auf. Schliesslich umfasst die Radialquerschnittsfläche Q' einen Flächenschwerpunkt 11, der bezüglich der Längsachse A einen Abstand und eine Lage aufweist. Sowohl Lage wie auch Abstand des Flächenschwerpunktes 11 ist für alle Flächeninhalte Q', die sich im ersten Azimutwinkelbereich 9 befinden. Der Schraubenkopf kann einen Antrieb, beispielsweise einen Torx-Antrieb (nicht gezeigt) enthalten. Dieser ist vorliegend zur Berechnung der Flächeninhalte nicht miteinbezogen worden. Stattdessen wurde der Antrieb gedanklich ausgefüllt und sein Flächeninhalt zum Kopf 4 gezählt. Ebenso ist aus Fig. 3a, wie auch aus den nachfolgenden Figuren 3b und 3c, ersichtlich, dass die Radialquerschnitte Q',Q" wegzusammenhängend und einfach zusammenhängend sind. Dies bedeutet, dass alle Punkte innerhalb der Fläche über einen zur Fläche gehörenden Weg miteinander verbunden werden können. Zusätzlich kann jeder geschlossene Weg innerhalb der Fläche auf einen Punkt zusammengezogen werden. Dies wäre bei einem Schraubenkopf, der eine Bohrung oder ein Loch enthielte, unter Umständen nicht möglich.

Fig. 3b zeigt eine Querschnittsansicht des Schraubenkopfes 4 der Figuren 2a-2c in der Ebene E'. Zwei Radialquerschnitte Q',Q" sind jeweils von der Umrandung des Kopfes 4, der Längsachse A und der Ebene 51 begrenzt und weisen Flächeninhalte auf. Die Radialquerschnitte Q',Q" sind in ihrer Form und im Flächeninhalt unterschiedlich. Der Radialquerschnitt Q" ist im zweiten Azimutwinkelbereich 10 angeordnet und befindet sich daher in einem Kopfbereich, der eine Kerbe 7 aufweist. Der Radialquerschnitt Q" weist daher einen kleineren Flächeninhalt auf als der Radialquerschnitt Q'. Der Radialquerschnitt Q' weist dagegen den gleichen Flächeninhalt wie auch die gleich Form wie die beiden Radialquerschnitte Q' aus der Fig. 3a auf, da sie sich im ersten Azimutwinkelbereich 9 befinden. Ebenso ist der Flächenschwerpunkt 11 des Radialquerschnittes Q' im gleichen Abstand und in der gleichen Lage zur Längsachse A angeordnet wie in Fig. 3a gezeigt.

Fig. 3c zeigt eine Querschnittsansicht des Schraubenkopfes 4 der Figuren 2a-2c in der Ebene E". Die Darstellung entspricht im Wesentlichen derjenigen von Fig. 3b, wobei aber die Radialquerschnitte Q' und Q" umgekehrt orientiert sind. In der vorliegenden Figur ist der Flächenschwerpunkt 11 des Radialquerschnittes Q" eingezeichnet. Dieser ist im zweiten Azimutwinkelbereich 10 variabel, kann also je nach Azimutwinkel relativ zur Längsachse A eine unterschiedliche Lage und einen unterschiedlichen Abstand zur Längsachse A aufweisen.

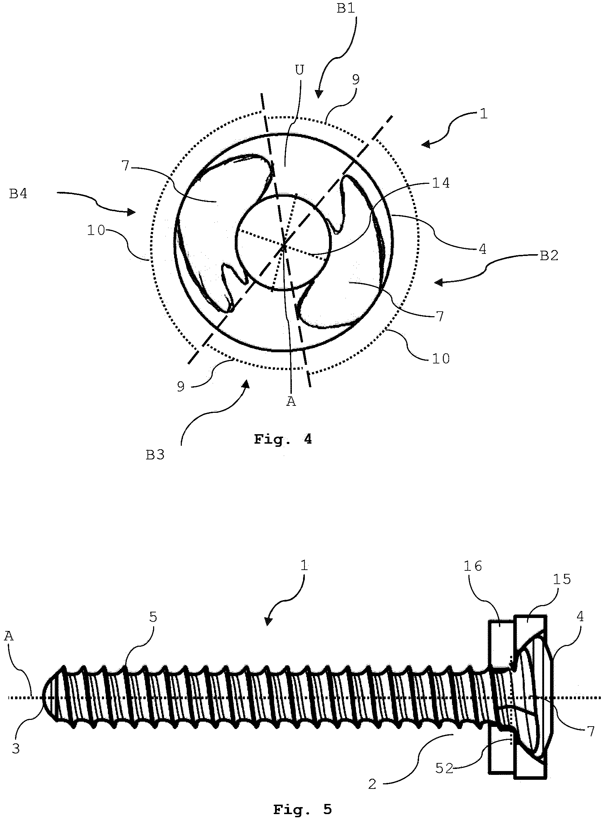

Fig. 4 zeigt einen Kopf 4 einer alternativen Ausführungsform einer Schraube 1 in einer Untersicht. Der Schraubenkopf 4 weist an seiner Unterseite U zwei Einkerbungen 7 auf. Ein solcher Schraubenkopf ist besonders geeignet, um mit mehrgängigen, insbesondere zweigängigen, Schrauben verwendet zu werden. Die Kopfbereiche, die eine Einkerbung 7 aufweisen, befinden sich im zweiten Azimutwinkelbereich 10. Der erste Azimutwinkelbereich 9 weist keine Einkerbungen 7 auf und ist daher spiegelsymmetrisch relativ zur Längsachse A ausgeführt, die vorliegend senkrecht zur Bildebene verläuft. Der erste Azimutwinkelbereich 9 und der zweite Azimutwinkelbereich 10 sind vorliegend in je zwei Abschnitte B1,B2,B3,B4 unterteilt, die vom jeweils anderen Azimutwinkelbereich 9,10 begrenzt sind und in gleichen Azimutwinkelabständen angeordnet sind. Als Referenzpunkt zur Bestimmung des Winkelabstandes wird vorliegend bevorzugt die Winkelhalbierende 14 der Begrenzungen der Abschnitte verwendet. Vorliegend beträgt daher der Winkelabstand zwischen den Abschnitten 90°. Vorliegend weist der erste Azimutwinkelbereich 9 gesamthaft einen Wert von 90° auf, der auf zwei Abschnitte von jeweils 45° verteilt ist. Wenn der gezeigte Schraubenkopf 4 mit einer Schraube mit einem zweigängigen Gewinde verwendet wird, weist der Kopf 4 ausserdem die gleiche Anzahl Abschnitte auf, wie das Gewinde Gänge aufweist. Ausserdem weist dann jeder Abschnitt des zweiten Azimutwinkelbereiches 10 genau eine Einkerbung 7 auf.

Fig. 5 zeigt eine Schraube 1 im eingeschraubten Zustand mit einer Knochenplatte 15 in einer dünnen Kortikalisschicht 16. Die Schraube 1 entspricht im Wesentlichen der in Fig. 1 gezeigten Schraube 1. Ebenso gezeigt ist die Ebene 52, die den Kopf 4 vom Schaft 2 trennt. Durch die Einkerbung 7 und das Gewinde 5, das sich bis in den Kopf 4 erstreckt, greift das Gewinde 5 über zumindest einen Teil der Dicke des Knochens 16, obwohl dieser dünn ist. Das Gewinde 5 erstreckt sich vorliegend bis in die Öffnung der Knochenplatte 15.

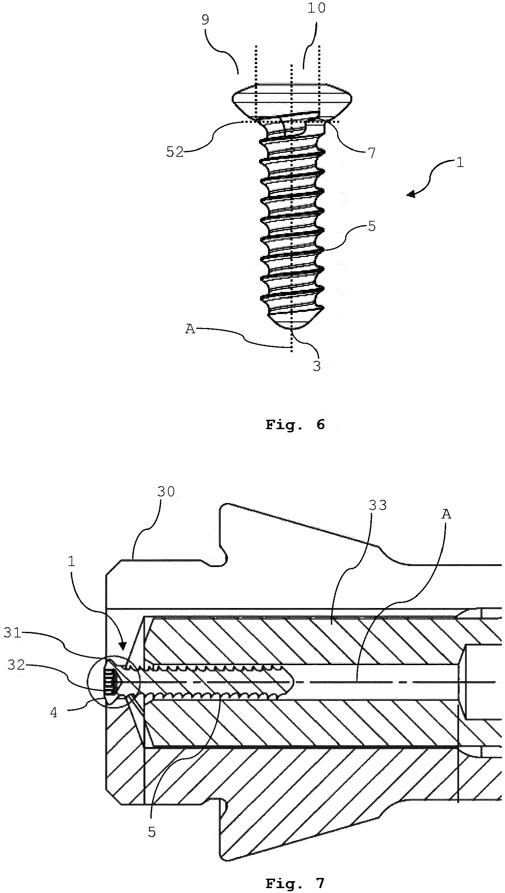

Fig. 6 zeigt eine alternative Ausführungsform einer Schraube 1, welche mit einem modifizierten Wirbelmesser ohne Überschneide erzeugt wurde. Die Kerbe 7 ist daher vorliegend kleiner ausgeführt als bei der Schraube 1 von Fig. 1, obwohl das Gewinde 5 im Wesentlichen dem Gewinde 5 aus Fig. 1 entspricht. Der erste Azimutwinkelbereich 9 weist keine Einkerbung 7 auf. Der zweite Azimutwinkelbereich 10, in dem die Einkerbung 7 liegt, weist einen Wert von 45° auf. Der erste Azimutwinkelbereich 9 weist daher einen Wert von 315° auf.

Fig. 7 zeigt schematisch einen Verfahrensschritt zur Herstellung einer Schraube 1. Vorliegend wurde mittels eines Wirbelmessers (nicht gezeigt) ein Gewinde 5 in ein stangenförmiges Rohlingsmaterial geschnitten. Im gezeigten Verfahrensschritt wird die Schraube 1 nach dem Einbringen des Gewindes 5 mittels einer Konturspannzange 30 am Kopf 4 gehalten. Zusätzlich verhindert eine Führungshülse 33, dass die Schraube 1 beim Öffnen der Konturspannzange 30 kippt. Die Konturspannzange 30 weist dafür eine Kontur 31 auf, die vorliegend so ausgeführt ist, dass sie eine Gegenkontur zum Kopf 4 der Schraube 1 bildet. Sie ist daher geeignet, um den Kopf 4 der Schraube 1 abzugreifen und diesen sicher zu halten. Es wäre aber denkbar, auch andere Konturen zu verwenden, die z.B. auf dem Kopf oder Teilen des Kopfes sowie Teilen des Schraubenschafts oder Gewindes abzugreifen. Insbesondere könnte auch nur ein Abschnitt des Kopfes 4 gehalten werden. Durch das Abgreifen der Schraube 1 am Kopf, wie es vorliegend gezeigt ist, wird ermöglicht, den Kopf 4 weiter zu bearbeiten, beispielsweise um einen Schraubenantrieb, vorliegend ein Torx-Antrieb 32, zu fertigen. Vorliegend ist der Verfahrensschritt mit einer Schraube 1 wie aus Fig. 1 oder Fig. 6 gezeigt, bei der sich das Gewinde bis in den Kopf erstreckt. Daher wäre ein Abgriff einer herkömmlichen Spannzange nicht möglich, da ein Halten am Gewinde 5 das Gewinde 5 beschädigen würde. Der gezeigte Verfahrensschritt ist deswegen besonders geeignet, um eine erfindungsgemässe Schraube herzustellen. Es ist für den Fachmann aber ersichtlich, dass der gezeigte Verfahrensschritt auch zur Herstellung einer herkömmlichen Schraube geeignet ist.

## Patentansprüche

1. Eine Schraube (1), bevorzugt eine Knochenschraube, umfassend einen Schaft (2) mit einer Spitze (3), einen Kopf (4) und ein Gewinde (5), sowie eine Längsachse (A) des Schaftes (2), und einer Schar von Radialquerschnittsflächen (Q) des Kopfes (4), die durch die Längsachse (A) der Schaftes (2) verlaufen, wobei die Lage jeder Radialquerschnittsfläche (Q) durch einen Azimutwinkel (6) in einer Ebene (E) senkrecht zur Längsachse (A) definiert ist, und wobei jede Radialquerschnittsfläche (Q) einen Flächeninhalt umfasst, welcher durch die Längsachse (A) und die Aussenfläche der Schraube (8) definiert wird,
**dadurch gekennzeichnet, dass**
das Gewinde (5) sich derart in den Kopf (4) erstreckt, so dass die Flächeninhalte der Radialquerschnittsflächen (Q') in einem ersten Azimutwinkelbereich (9) konstant sind und die Flächeninhalte der Radialquerschnittsflächen (Q") in einem zweiten, vom ersten verschiedenen Azimutwinkelbereich (10) einen abweichenden, bevorzugt kleineren, Wert aufweisen als die Flächeninhalte im ersten Azimutwinkelbereich (9), wobei der erste Azimutwinkelbereich (9) höchstens 350°, bevorzugt höchstens 345°, beträgt.

2. Schraube (1) gemäss Anspruch 1, wobei der erste und der zweite Azimutwinkelbereich (Q', Q") in jeweils mindestens zwei, bevorzugt zwei oder drei, Abschnitte unterteilt ist, die in gleichen Azimutwinkelabständen verteilt sind.

3. Schraube (1) gemäss Anspruch 2, wobei der erste Azimutwinkelbereich (9) höchstens 330°, bevorzugt höchstens 305°, beträgt.

4. Schraube (1) gemäss einem der Ansprüche 1 bis 3, wobei das Gewinde (5) mehrgängig, bevorzugt zwei- oder dreigängig ist.

5. Schraube (1) gemäss einem der vorhergehenden Ansprüche, wobei der Kopf im zweiten Azimutwinkelbereich (10) mindestens eine Einkerbung (7), welche durch die Fortsetzung des Gewindes (5) gebildet ist, aufweist.

6. Schraube (1) gemäss Anspruch 5 sowie einem der Ansprüche 2 bis 4, wobei der Kopf (4) eine Anzahl Einkerbungen (7), welche durch die Fortsetzung des Gewindes (5) gebildet sind, aufweist, die der Anzahl Abschnitte des Azimutwinkelbereiches entspricht.

7. Schraube (1) gemäss Anspruch 6, wobei jeder Abschnitt des Azimutwinkelbereiches eine Einkerbung (7), welche durch die Fortsetzung des Gewindes (5) gebildet ist, aufweist.

8. Schraube (1) gemäss einem der Ansprüche 5 bis 7, wobei die mindestens eine Einkerbung (7), welche durch die Fortsetzung des Gewindes (5) gebildet ist, an einer Unterseite (U) des Kopfes (4) im Bereich des Gewindeauslaufs angeordnet ist.

9. Schraube (1) gemäss einem der vorhergehenden Ansprüche, wobei die Radialquerschnittsflächen (Q) einen Flächenschwerpunkt (11) aufweisen, und der Flächenschwerpunkt (11) der Radialquerschnittsflächen im ersten Azimutwinkelbereich (Q) jeweils den gleichen Abstand zur Längsachse aufweist und bevorzugt eine identische Lage entlang der Längsachse (A) aufweist.

10. Schraube (1) gemäss einem der vorhergehenden Ansprüche, wobei die Schraube so ausgebildet, insbesondere geformt ist, so dass jede Radialquerschnittsfläche (Q', Q") zusammenhängend, insbesondere wegzusammenhängend, ist.

11. Schraube (1) gemäss einem der vorhergehenden Ansprüche, wobei ein erster Normalquerschnitt (13) in einer Ebene senkrecht zur Längsachse (A) im Kopfbereich (4) mindestens teilweise nicht kreisrund ist.

12. Schraube (1) gemäss Anspruch 11, wobei ein zweiter Normalquerschnitt (12) kreisförmig ist und parallel zum ersten Normalquerschnitt (12) ist, und der erste Normalquerschnitt (12) entlang der Längsachse (A) der Schraube (1) zwischen der Spitze (3) der Schraube (1) und dem zweiten Normalquerschnitt (13) angeordnet ist.

13. System umfassend eine Knochenplatte und eine Knochenschraube (1), bevorzugt eine Knochenschraube nach einem der vorhergehenden Ansprüche, wobei die Knochenplatte mindestens eine Öffnung zum Einsetzen der Knochenschraube umfasst, und wobei die Knochenschraube einen Kopf, einen Schaft und ein durchgehendes Gewinde umfasst, wobei die Knochenplatte bevorzugt eine Dicke aufweist, die höchstens der Länge des Kopfes der Schraube entlang einer Längsachse der Schraube entspricht, **dadurch gekennzeichnet, dass** die Knochenschraube derart in die Öffnung der Knochenplatte einsetzbar ist, dass der Kopf in Kontakt mit der Knochenplatte ist, insbesondere auf der Knochenplatte aufliegt, und das Gewinde sich zumindest teilweise in die Öffnung hinein erstreckt.

14. Verfahren zum Herstellen einer Schraube (1), bevorzugt einer Knochenschraube, insbesondere eine Knochenschraube nach einem der Ansprüche 1 bis 12, mit einem Werkzeug, bevorzugt ausgewählt aus einer Gruppe umfassend Wirbelmesser und einem Gewindefräser, umfassend die Schritte:
- Bereitstellen eines Rohlingmaterials mit einer im Wesentlichen zylindrischen Form,
- Schneiden eines Gewindes (5) entlang einer Längsachse (A) der zylindrischen Form mit dem Werkzeug
- Formen eines Kopfes (4), insbesondere durch Drehen, **dadurch gekennzeichnet, dass** das Rohlingmaterial mittels einer Konturspannzange (30) am Kopf (4) abgegriffen wird, wobei die Konturspannzange (30) eine Kontur (31) aufweist, die im abgegriffenen Zustand mit einer Gegenkontur des Kopfes (4) in Wirkverbindung steht.

15. Verfahren gemäss Anspruch 14, wobei die Kontur (31) der Konturspannzange (30) im Wesentlichen mindestens teilweise komplementär zu der Gegenkontur des Kopfes (4) der Schraube (1) ist.

16. Verfahren gemäss einem der Ansprüche 14 oder 15, wobei das Gewinde mit Hilfe des Werkzeuges mindestens so nahe an den Kopf geschnitten wird, dass das Werkzeug eine Einkerbung an einer Unterseite des Kopfes bildet.

17. Verfahren gemäss einem der Ansprüche 14 bis 16, wobei das Werkzeug eine Wirbelplatte aufweist, die in einer Schneidrichtung keine Überschneide umfasst.

18. Verfahren gemäss einem der Ansprüche 14 bis 17, wobei nach dem Schneiden des Gewindes mit dem Werkzeug der Kopf mittels einer CNC Langdrehmaschine weiterbearbeitet wird.
